# FASCICULE DE BREVET EUROPEEN

(11) **EP 1 052 987 B1**
(45) Date de publication et mention de la délivrance du brevet: **06.02.2002**
(21) Numéro de dépôt: 98955688.1
(22) Date de dépôt: 18.11.1998
(51) Int. Cl.: A61K 31/295, A61K 9/00

(54) **COMPRIMES A CROQUER OU A SUCER, COMPRENANT COMME PRINCIPE ACTIF DU FER**
EISEN ALS WIRKSTOFF ENTHALTENDE KAU- ODER LUTSCHPRESSLINGE
TABLETS TO BE CRUNCHED OR SUCKED, COMPRISING IRON AS ACTIVE PRINCIPLE

(30) Priorité: 03.02.1998 FR 9801223
(43) Date de publication de la demande: 22.11.2000
(73) Titulaire: LABORATOIRE INNOTHERA Société Anonyme, 94111 Arcueil (FR)
(72) Inventeur: MEIGNANT, Catherine, F-75010 Paris (FR); VERDIER, Stéphanie, F-75005 Paris (FR)
(74) Mandataire: Dupuis-Latour, Dominique
(86) Numéro de dépôt international: FR9802462
(87) Numéro de publication internationale: WO9939708

(56) Documents cités:
- EP-A- 0 737 473
- GB-A- 2 195 890
- GB-A- 2 195 891
- US-A- 5 409 905

## Description

L'invention concerne une spécialité pharmaceutique sous forme galénique unitaire de comprimés à croquer ou à sucer comprenant comme principe actif du fer élément.

Une telle spécialité est indiquée dans la prévention ou la correction des troubles en rapport avec une carence ou un déséquilibre en fer. Ces troubles sont traités par apport de fer élément sous forme de sels ferreux (Fe²⁺) ou ferriques (Fe³⁺), ces derniers étant toutefois moins employés.

Lorsque ces sels de fer sont administrés *per os,* une difficulté tient à leur goût désagréable lorsqu'ils sont aux doses procurant les effets thérapeutiques recherchés, à savoir des doses de l'ordre de plusieurs milligrammes ou plusieurs dizaines de milligrammes par comprimé (on exclura le cas des compléments alimentaires multiples et autres produits de supplémentation minéralo-vitaminique, dont la teneur en fer est très faible et n'a donc pas d'incidence gustative).

Pour cette raison, les spécialités comprenant comme principe actif un sel de fer sont généralement présentés sous forme de comprimé enrobé (dragéifié) ou pelliculé, de sorte que la problématique du goût désagréable du sel de fer ne se pose pas pour ces spécialités.

L'un des buts de la présente invention est de proposer une formulation particulière sous forme de comprimé à croquer ou à sucer pour une spécialité contenant un sel de fer, qui soit galéniquement acceptable, fabricable industriellement, stable et, surtout, de goût acceptable.

À cet effet, la spécialité de l'invention comprend un sel de fer, au moins deux polyols, un agent liant, un agent lubrifiant et un agent aromatisant.

Selon diverses caractéristiques subsidiaires avantageuses :
― la proportion de l'un des polyols à lui seul est d'au moins 10 %, de préférence au moins 20 %, de la masse totale du comprimé, et la proportion globale des polyols est d'au moins 50 %, de préférence au moins 75 %, de la masse totale du comprimé ;
― l'un des deux polyols est le xylitol, en proportion d'au moins 10 %, de préférence au moins 20 %, de la masse totale du comprimé ;
― la spécialité comprenant éventuellement trois polyols, le polyol autre que le xylitol est le sorbitol et/ou le mannitol ;
― l'agent liant est la cellulose microcristalline;
― l'agent lubrifiant est choisi dans le groupe comprenant l'acide stéarique micronisé, le talc et leurs mélanges ;
― le sel de fer est soit un sel ferreux, tel que le sulfate de fer, le succinate de fer, le gluconate de fer, le glycérophosphate de fer, le glucoheptonate de fer, le *dl*-aspartate de fer, le fumarate de fer, le pyrophosphate ferreux et l'oxyde ferreux, soit un sel ferrique tel que le citrate d'ammonium ferrique, le chlorure ferrique, le citrate ferrique, le pyrophosphate ferrique et l'édétate ferrique ; la dose de fer est d'au moins 5 mg, de préférence 10 à 40 mg de fer élément par comprimé ;
― la formule générale de la spécialité est : sulfate ferreux, quantité correspondant à fer élément 5 à 15 mg ; xylitol, 550 mg (± 10 %) ; mannitol, 300 à 700 mg (± 10 %) ; cellulose microcristalline, 100 mg (± 10 %) ; arôme (q. s. aromatisation), = 230 mg ; acide stéarique, 65 mg (± 10 %) ; talc, 10 mg ; sorbitol, q. s. (± 10 %) un comprimé de 1600 à 2300 mg .

On va décrire un exemple préférentiel, mais non limitatif, de mise en oeuvre de l'invention.

On va tout d'abord détailler chacun des constituants de la formulation, à savoir : sel de fer, polyols, liant, lubrifiant et aromatisant.
1°) sel de fer : le sel utilisé est de préférence un sel ferreux.
   Ce sel peut notamment être du sulfate ferreux anhydre, se présentant sous forme d'une poudre fine verdâtre. Pour une dose de Fe²⁺ de 10 mg par comprimé, valeur typique, le fer élément sera apporté par 27,2 mg de sulfate de fer anhydre. Cette dose de 10 mg de Fe²⁺ par comprimé n'est bien entendu pas limitative, et d'autres doses pourront bien entendu être envisagées, allant généralement, mais de façon ici encore non limitative, de 5 à 15 mg de fer élément par comprimé, correspondant à des quantités de 13,6 à 40,8 mg de sulfate ferreux anhydre lorsque ce sel est choisi.
   D'autres sels ferreux peuvent être employés, tels que le succinate de fer, le gluconate de fer, le glycérophosphate de fer, le glucoheptonate de fer, le *dl*-aspartate de fer, le fumarate de fer, le pyrophosphate ferreux ou l'oxyde ferreux, avec bien entendu à chaque fois une adaptation des quantités du sel de fer pour aboutir à la dose de fer élément recherchée.
   En variante,il est possible d'employer aussi des sels ferriques tels que le citrate d'ammonium ferrique, le chlorure ferrique, le citrate ferrique, le pyrophosphate ferrique ou l'édétate ferrique.
2°) polyols: l'une des caractéristiques de la formulation de l'invention consiste en l'emploi d'au moins deux polyols différents, de préférence trois polyols, employés comme diluants, agents de compression, édulcorants et, surtout, agents de masquage du goût du fer.
   Les polyols utilisés préférentiellement sont le sorbitol, le mannitol et le xylitol.
   Comme indiqué, ces trois polyols sont de préférence utilisés conjointement ; si deux seulement sont utilisés, on associe préférentiellement sorbitol et xylitol, ou bien mannitol et xylitol.
   Pour aboutir à l'effet principal recherché (le masquage du goût du fer), l'un des polyols, de préférence le xylitol, doit représenter à lui seul au moins 10 %, de préférence au moins 20 %, de la masse totale du comprimé, la proportion des deux ou trois polyols ensemble étant d'au moins 50 %, de préférence au moins 75 % de la masse totale .
   Le sorbitol sera par exemple du type *Neosorb P 60 W*®, Roquette. Ce polyol se présente sous la forme d'une poudre granuleuse blanche, d'un diamètre moyen de 200 µm et possède d'excellentes propriétés liantes en compression. Son pouvoir sucrant est de 70 % de celui du saccharose, il est acariogène et peu calorique (2,4 Kcal/g contre 4 Kcal/g pour le saccharose).
   Le mannitol sera par exemple du type *Pearlitol SD 200*®, Roquette. Ce polyol se présente sous la forme d'une poudre blanche, inodore, d'un diamètre moyen d'environ 170 µm. Son pouvoir sucrant est de 40 % de celui du saccharose, il est acariogène et également peu calorique (2,4 Kcal/g).
   Le xylitol sera par exemple du type *Xylitab 300*®, Finnsugar. Ce polyol se présente sous la forme d'une poudre granuleuse cristalline blanche, d'un diamètre moyen de 250 µm. Il est de saveur sucrée (équivalente à celle du saccharose) procurant une agréable sensation de fraîcheur dans la bouche, il est acariogène et également très peu calorique (2,4 Kcal/g). Ce xylitol particulier possède par ailleurs des propriétés de comprimabilité supérieures à celles du xylitol standard. La sensation de fraîcheur combinée à l'apport sucré rend particulièrement intéressant le xylitol comme agent de masquage du goût du fer.
3°) agent liant : on peut utiliser notamment de la cellulose microcristalline, par exemple de type *Avicel*® , qui permet également d'améliorer l'écoulement du produit pulvérulent lors de la compression.
4°) arôme : il est choisi parmi des additifs classiques en fonction du goût que l'on souhaite donner à la préparation ; on peut par exemple utiliser un arôme mûre (SBI).
5°) lubrifiant: pour améliorer la fabrication, on peut avantageusement utiliser une association de lubrifiants, par exemple une combinaison d'acide stéarique micronisé et de talc. D'autres lubrifiants, en eux-mêmes classiques, sont bien entendu envisageables, par exemple le stéarate de magnésium. Le lubrifiant se présente sous la forme d'une poudre fine, blanchâtre, permettant d'éviter le phénomène de grippage au niveau des matrices des presses lors de la compression finale du mélange.

Pour la fabrication, le protocole opératoire est le suivant : prémélange du sulfate de fer avec du sorbitol, les proportions respectives étant choisies pour avoir un volume de sorbitol environ double de celui du sulfate de fer ; mélange de la composition intermédiaire ainsi prémélangée avec les quantités requises de xylitol, mannitol, le reste du sorbitol, cellulose microcristalline, arôme et talc ; addition de l'acide stéarique ; enfin, compression sur une presse à comprimés, à 15-20 kN environ.

Une formule générale préférentielle est la suivante :

| | |
|---|---|
| Sulfate ferreux : qté. correspondant à fer élément | 5 à 15 mg |
| Xylitol | 550 mg (± 10 %) |
| Mannitol | 300 à 700 mg (± 10 %) |
| Cellulose microcristalline | 100 mg (± 10 %) |
| Arôme | (q. s. aromatisation) ≈ 230 mg |
| Acide stéarique | 65 mg (± 10 %) |
| Talc | 10 mg |
| Sorbitol | q. s. un comprimé de 1600 à 2300 mg (± 10 %) |

## Revendications

1. Une spécialité pharmaceutique sous forme galénique unitaire de comprimés à croquer ou à sucer comprenant comme principe actif du fer élément, **caractérisée en ce qu'**elle comprend :
― un sel de fer,
― au moins deux polyols,
― un agent liant,
― un agent lubrifiant et
― un agent aromatisant.

2. La spécialité de la revendication 1, dans laquelle la proportion de l'un des polyols à lui seul est d'au moins 10 %, de préférence au moins 20 %, de la masse totale du comprimé.

3. La spécialité de la revendication 1, dans laquelle la proportion globale des polyols est d'au moins 50 %, de préférence au moins 75 %, de la masse totale du comprimé.

4. La spécialité de la revendication 1, dans laquelle l'un des deux polyols est le xylitol.

5. La spécialité des revendications 2 et 4 prises en combinaison, dans laquelle la proportion du xylitol est d'au moins 10 %, de préférence au moins 20 %, de la masse totale du comprimé.

6. La spécialité de la revendication 1, comprenant trois polyols.

7. La spécialité de l'une des revendications 4 et 6, dans lequel l'autre polyol est le sorbitol et/ou le mannitol.

8. La spécialité de la revendication 1, dans laquelle l'agent liant est la cellulose microcristalline.

9. La spécialité de la revendication 1, dans laquelle l'agent lubrifiant est choisi dans le groupe comprenant l'acide stéarique micronisé, le talc et leurs mélanges.

10. La spécialité de la revendication 1, dans laquelle le sel de fer est un sel ferreux.

11. La spécialité de la revendication 10, dans laquelle le sel de fer est choisi parmi le sulfate de fer, le succinate de fer, le gluconate de fer, le glycérophosphate de fer, le glucoheptonate de fer, le *dl*-aspartate de fer, le fumarate de fer, le pyrophosphate ferreux et l'oxyde ferreux.

12. La spécialité de la revendication 1, dans laquelle le sel de fer est un sel ferrique.

13. La spécialité de la revendication 12, dans laquelle le sel de fer est choisi parmi le citrate d'ammonium ferrique, le chlorure ferrique, le citrate ferrique, le pyrophosphate ferrique et l'édétate ferrique.

14. La spécialité de la revendication 1, dans laquelle la dose de fer est d'au moins 5 mg, de préférence 10 à 40 mg de fer élément par comprimé.

15. La spécialité de la revendication 1, de formule générale :
| | |
|---|---|
| Sulfate ferreux : qté. correspondant à fer élément | 5 à 15 mg |
| Xylitol | 550 mg (± 10 %) |
| Mannitol | 300 à 700 mg (± 10 %) |
| Cellulose microcristalline | 100 mg (± 10 %) |
| Arôme | (q. s. aromatisation) ≈ 230 mg |
| Acide stéarique | 65 mg (± 10 %) |
| Talc | 10 mg |
| Sorbitol | q. s. un comprimé de 1600 à 2300 mg (± 10 %) |

## Patentansprüche

1. Pharmazeutische Spezialität als galenische Einheit in Form von Tabletten zum Kauen oder Lutschen, die als aktiven Wirkstoff einen Eisenbestandteil enthalten, dadurch charakterisiert, daß sie enthalten:
- ein Eisensalz,
- wenigstens zwei Polyole,
- ein Bindemittel,
- ein Gleitmittel und
- ein aromatisierendes Agens.

2. Spezialität nach Anspruch 1, wobei die Menge einer der Polyole für sich allein wenigstens 10 %, vorzugsweise wenigstens 20 % der Gesamtmenge der Tablette beträgt.

3. Spezialität nach Anspruch 1, wobei die Gesamtmenge der Polyole wenigstens 50 %, vorzugsweise wenigstens 75 % der Gesamtmenge der Tablette beträgt.

4. Spezialität nach Anspruch 1, wobei einer der zwei Polyole Xylit ist.

5. Spezialität nach den Ansprüchen 2 und 4 in Kombination, wobei die Menge an Xylit wenigstens 10 %, vorzugsweise wenigstens 20 % der Gesamtmenge der Tablette beträgt.

6. Spezialität nach Anspruch 1, enthaltend drei Polyole.

7. Spezialität nach einem der Ansprüche 4 und 6, wobei das andere Polyol Sorbit und/oder Mannit ist.

8. Spezialität nach Anspruch 1, wobei das Bindemittel mikrokristalline Cellulose ist.

9. Spezialität nach Anspruch 1, wobei das Gleitmittel ausgewählt ist aus der Gruppe umfassend pulverisierte Stearinsäure, Talkum und deren Gemische.

10. Spezialität nach Anspruch 1, wobei das Eisensalz ein Eisen(II)-Salz ist.

11. Spezialität nach Anspruch 10, wobei das Eisensalz ausgewählt ist unter Eisensulfat, Eisensuccinat, Eisengluconat, Eisenglycerolphosphat, Eisenglycoheptansäure, Eisen *dl*-Aspartat, Eisenfumarat, Eisen(II)-pyrophosphat und Eisen(II)-oxid.

12. Spezialität nach Anspruch 1, wobei das Eisensalz ein Eisen(III)-Salz ist.

13. Spezialität nach Anspruch 12, wobei das Eisensalz ausgewählt ist aus Eisen(III)-ammoniumcitrat, Eisen(III)-chlorid, Eisen(III)-citrat, Eisen(III)-pyrophosphat und Eisen(III)-ethylendiamintetraacetat.

14. Spezialität nach Anspruch 1, wobei die Eisendosis wenigstens 5 mg, vorzugsweise 10 bis 40 mg, bezogen auf Eisen pro Tablette beträgt.

15. Spezialität nach Anspruch 1 der allgemeinen Formulierung:
| | |
|---|---|
| Eisen(II)-sulfat: Entsprechende Menge an Eisen | 5 bis 15 mg |
| Xylit | 550 mg (±10%) |
| Mannit | 300 bis 700 mg (± 10 %) |
| mikrokristalline Cellulose | 100 mg (± 10 %) |
| Aroma (ausreichende Menge zur Aromatisierung) | ≈ 230 mg |
| Stearinsäure | 65 mg (± 10%) |
| Talkum | 10 mg |
| Sorbit: ausreichende Menge für eine Tablette von 1600 bis 2300 mg (± 10 %) | |

## Claims

1. A pharmaceutical medicine in unit galenical formulation of tablets for chewing or sucking and having elemental iron as its active principle, the medicine being **characterized in that** it comprises:
· an iron salt;
· not less than two polyols;
· a binding agent;
· a lubricating agent; and
· a flavoring agent.

2. The medicine of claim 1, in which the proportion of one of the polyols on its own is not less than 10%, and preferably not less than 20% of the total mass of the tablet.

3. The medicine of claim 1, in which the overall proportion of polyols is not less than 50%, preferably not less than 75%, of the total mass of the tablet.

4. The medicine of claim 1, in which one of the two polyols is xylitol.

5. The medicine of claims 2 and 4 taken in combination, in which the proportion of xylitol is not less than 10%, preferably not less than 20%, of the total mass of the tablet.

6. The medicine of claim 1, having three polyols.

7. The medicine of claim 4 or 6, in which the other polyol is sorbitol and/or mannitol.

8. The medicine of claim 1, in which the binding agent is microcrystalline cellulose.

9. The medicine of claim 1, in which the lubricating agent is selected from the group comprising micronized stearic acid, talc, and mixtures thereof.

10. The medicine of claim 1, in which the iron salt is a ferrous salt.

11. The medicine of claim 10, in which the iron salt is selected from iron sulfate, iron succinate, iron gluconate, iron glycerophosphate, iron glucoheptonate, iron *dl*-asparate, iron fumarate, iron pyrophosphate, and iron oxide.

12. The medicine according to claim 1, in which the iron salt is a ferric salt.

13. The medicine of claim 12, in which the iron salt is selected from ferric ammonium citrate, ferric chloride, ferric citrate, ferric pyrophosphate, and ferric edetate.

14. The medicine of claim 1, in which the dose of iron is not less than 5 mg, and preferably 10 mg to 40 mg of elemental iron per tablet.

15. The medicine of claim 1, having the following general formula:
· ferrous sulfate: quantity corresponding to elemental iron: 5 mg to 15 mg;
· xylitol: 550 mg (±10%);
· mannitol; 300 mg to 700 mg (±10%);
· microcrystalline cellulose: 100 mg (±10%);
· flavoring (sufficient to produce flavor): ≈ 230 mg;
· stearic acid: 65 mg (±10%);
· talc: 10 mg; and
· sorbitol (sufficient to make a tablet weighing 1600 mg to 2300 mg (±10%).
